# EUROPEAN PATENT SPECIFICATION

(11) **EP 2 050 823 B1**
(45) Date of publication and mention of the grant of the patent: **05.09.2012**
(21) Application number: 07803637.3
(22) Date of filing: 20.07.2007
(51) Int. Cl.: C12P 7/64, C10G 3/00, C11C 3/10

(54) **METHOD FOR PRODUCING BIODIESEL USING PORCINE PANCREATIC LIPASE AS AN ENZYMATIC BIOCATALYST**
VERFAHREN ZUR HERSTELLUNG VON BIODIESEL UNTER VERWENDUNG VON LIPASE AUS SCHWEINEPANKREAS ALS ENZYMATISCHEM BIOKATALYSATOR
PROCÉDÉ DE PRODUCTION DE BIODIESEL DANS LEQUEL DE LA LIPASE PANCRÉATIQUE DE PORC EST UTILISÉE COMME BIOCATALYSEUR ENZYMATIQUE

(30) Priority: 21.07.2006 ES 200601948
(43) Date of publication of application: 22.04.2009
(73) Proprietor: Universidad de Cordoba, 14071 Cordoba (ES)
(72) Inventor: LUNA MARTINEZ, Diego, 14071 Córdoba (ES); BAUTISTA RUBIO, Felipa María, 14071 Córdoba (ES); CABALLERO MARTÍN, Verónica, 14071 Córdoba (ES); CAMPELO PÉREZ, Juan Manuel, 14071 Córdoba (ES); MARINAS RUBIO, José María, 14071 Córdoba (ES); ROMERO REYES, Antonio Ángel, 14071 Córdoba (ES)
(74) Representative: Temino Ceniceros, Ignacio
(86) International application number: PCT/ES2007/000450
(87) International publication number: WO 2008/009772

(56) References cited:
- WO-A1-00/56589
- WO-A1-2004/081158
- WO-A1-2005/075615
- WO-A1-2006/050589
- WO-A2-2006/077023
- FR-A1- 2 824 075
- DESAI P D ET AL: "Alcoholysis of salicornia oil using free and covalently bound lipase onto chitosan beads" FOOD CHEMISTRY, ELSEVIER LTD, NL LNKD- DOI:10.1016/J.FOODCHEM.2004.12.030, vol. 95, no. 2, 1 March 2006 (2006-03-01), pages 193-199, XP025130211 ISSN: 0308-8146 [retrieved on 2006-03-01]
- SHAH ET AL.: 'Biodiesthe preparation by lipase catalyzed transesterification of Jatropha oil' ENERGY & FUELS, AMERICAN CHEMIAL SOCIETY vol. 18, 2004, pages 154 - 159, XP008131533
- YESILOGLU ET AL.: 'Immobilized lipase catalyzed ethanolysis of sunflower oil' JOURNAL OF THE AMERICAN OIL CHEMISTS' SOCIETY vol. 81, 2004, pages 157 - 160, XP008131147

## Description

### TECHNICAL FIELD OF THE INVENTION

The present invention falls under the field of fuel production based on organic materials, specifically, oils or fats.

### STATE OF THE ART PRIOR TO THE INVENTION

The unceasing increase in petroleum prices have given rise to the fact that since more than 20 years ago, biodiesel (a mixture of methyl or ethyl esters obtained through the transesterification of oils or fats) has been increasingly in use in very different countries, more recently, including Spain, where it is used in admixture with conventional fossil diesel (30-36%). Indeed, the world market for this fuel type is undergoing continuous growth, basically due to the big subsidies it receives, owing to its environmental innocuity, since it does not count for the purposes of computing CO₂ (Kyoto Protocol), and, moreover, significantly reduces the level of particles generated by fossil fuels when these are mixed with them.

The transesterification reaction of oils and fats applied to the process of obtaining biodiesel may be carried out in an acid or basic medium, as well as through the action of specific enzymes, such as lipases. In this sense, operating in a basic medium implies significant advantages with respect to the application of acid catalysis, since the reaction kinetics are more favourable in a basic medium (x 4000), making it possible to operate with smaller amounts of alcohol at normal atmospheric pressure. Moreover, in a homogenous medium, alkalines are less corrosive than acid dissolutions. This last aspect is not to be considered if heterogeneous catalysts are used, but from the results published by very different authors, it is observed that comparatively higher energetic conditions are usually needed in the end (pressures superior to 5 atm and high temperatures, 100°C) when acid solids are used as catalysts.

At present, almost all industrial processes use different alkaline metals in homogenous phase as catalysts, fundamentally NaOH or KOH, since this is what yields better performance under conditions of relatively lower energy (70°C and alcohol/oil 2-4/1). Nonetheless, this presents certain weak points associated to its homogenous character, which not only imply the loss of the catalyst (it is used only once), but also the need to eliminate alkaline residues from the biodiesel before it is used as fuel. This implies several additional washing steps involving the use of a large amount of water that has to be treated afterwards in order to eliminate the alkaline residues contained in it after the washing process. That inevitably increases the end costs of production. Moreover, the quality of the glycerine obtained as by-product of the principal reaction for obtaining the mixture of methyl (or ethyl) esters that make up the biodiesel is reduced by the serious contamination of alkaline residues that are very hard to eliminate.

It is for this reason that heterogeneous procedures based on alkaline earth metals in the form of oxides and/or carbonates are being developed. Research focuses on CaO, SrO, BaO, MgO, CaCO₃, and on highly diverse mixtures of these, or even incorporating alkaline metals such as K₂O, Na₂CO₃, K₂CO₃, KHCO₃, etc. In general, it is possible to operate in heterogeneous phase and achieve better-quality biodiesel and glycerine while at the same time simplifying the process by eliminating washing operations. In return, a significant drop in catalyst activity is always obtained, making it mandatory to operate under conditions of higher temperature and pressure. For the time being, the most promising results are those described with CaCO₃, since these involve a low-cost material that may be reused for several weeks [J.G. Suppes, K. Buchwinkel, S. Lucas, J.B. Botts, M.H. Mason, J.A. Heppert, J. Am. Oil Chem. Soc, 78 (2001) 139]. This notwithstanding, it is necessary to operate at high pressures and temperatures (240-269° C), with a high proportion of alcohol, 19/1 alcohol/oil, which, for the time being, rules out application on an industrial scale.

On another hand, lipases from diverse microorganisms have been widely investigated in this process, since they produce the specific enzymes that carry out hydrolysis or synthesis reactions on the ester bonds of triglycerides in living beings. Hence, lipases, in comparison with any of the methods described, solely offer advantages: since operating conditions are much milder, temperatures within the range of 30-50°C, pH 6-9 and normal atmospheric pressure. Moreover, it is not necessary to eliminate impurities of any sort (whether acid or alkaline), simplifying phase separation, obtaining high-grade biodiesel and glycerine appropriate for pharmaceutical use, and thus reducing the environmental impact of the process. [W.H. Wu, T.A. Foglia, W.N. Marmer, J.G. Phillips, J. Am. Oil. Chem. Soc., 76 (1999) 517]. In addition, its application is particularly appropriate for oils containing a high proportion of free fatty acids, as is the case of palm oil, used oil (those oils that may be used without pre-treatment), or alcohols with longer and/or more highly branched chains than methanol, such as ethanol, isopropanol, etc., which present greater difficulty of transesterification than methanol with conventional acid or basic catalysis.

The principal and most decisive inconvenience that enzymes in general, and thus lipases, present with regard to their application on a commercial scale is their elevated cost. Hence, the current consensus with respect to the industrial applicability of enzymes necessarily implies their attachment to or immobilization on any type of support that is compatible with the process to which they are to be applied; i.e., their "heterogeneization", such that their high cost is compensated by their reuse. Following immobilization, an increase in the activity and stability of enzymes with respect to their homogenous behaviour has been described [M. Iso, B. Chen, M. Eguchi, T. Kudo, S. Sherestha, J. Mil. Catal., 16 (2001) 53].

In this sense, the results described are very variable, whereby the compatibility of the reaction medium with the immobilization or attachment procedure and the support type plays a primordial role. Hence, there are descriptions, or even commercialized forms of diverse enzymatic preparations using resin supports for anionic exchange or even diverse organic polymers of the polyethylene or polystyrene type that yield very good results in aqueous media, not only because these are natural substrates for the enzymes, but mainly because their operation with organic solvents - as occurs in the specific case of biodiesel - produces effects of interaction (or attack), with enzyme swelling (deformation) and/or leaching (loss). This is the reason why there are descriptions of different attempts to apply inorganic supports, normally silica, in which immobilization is usually carried out by sol-gel confinement, since generating a covalent bond between the inorganic support and some functional group of the enzyme protein is more complicated than with a functionalized organic support (polystyrene, for example).

This notwithstanding, Spanish application P-9601847 publishes a procedure for the covalent immobilization of enzymes on inorganic supports that has been successfully applied to the immobilization of triacylglycerol lipase (EC 3.1.1.3), or pig pancreatic lipase (PPL) [F.M. Bautista, M.C. Bravo, J.M. Campelo, A. Garcia, D. Luna, J.M. Marinas, A.A. Romero, J. Chem. Technol. Biotechnol, 72 (1998) 249].

According to the results cited, the covalent immobilization of several enzymes, including a lipase on an inorganic support, is feasible, obtaining good results in terms of catalyst activity in all cases, particularly as regards the reuse of the immobilized enzymes. In fact, recently published results [A. Macario, G. Giordano, V. Calabro, A. Parise, D. Luna, V. Caballero, J.M. Campelo, J.M. Marinas, International Symposium on Environmental Biocatalysis, p. 8, Cordoba, 23-26 April 2006] indicate excellent prospects for obtaining biodiesel from olive oil using a commercial lipase, Rhizomucor Miehei (RML), covalently anchored onto sepiolite by applying the method of immobilization referred to.

Nonetheless, to date the price of the enzyme has rendered efforts to apply the enzymatic procedure (both using free enzymes and enzymes immobilized by diverse methods) infeasible on an industrial scale. Table 1 reflects some representative publications on the different types of lipases researched in the production of biodiesel (either in free or attached form) up to now, and the estimated price (as per the Sigma 2006 catalogue) of 10⁶ units of enzymatic activity (U), in which 1 U represents the amount of lipase necessary to hydrolyze 1 microequivalent of olive oil in 1 hour at 37° C and pH values in the range of 7-8, depending on the enzyme type.

In general, oils of vegetable and animal origin are used (soy, sunflower, palm, coconut, seed oil, grease, fish oil, etc.) including oils already used and diverse short-chain alcohols: methanol, ethanol, 1- and 2-propanol, 1- and 2-butanol and others such as 2-ethyl-1-hexanol. In the majority of cases, a conversion of > 90% is obtained, operating at temperatures in the range of 35-50° C, with reaction times of 12 to 70 hours, and alcohol/oil ratios of 3/l or higher. However, in no case has the application of "pig pancreatic lipase" (PPL) to any transesterification process related to the production of biodiesel been previously described.

Pig pancreatic lipase (PPL), "triacylglycerol lipase (EC 3.1.1.3)", is a biomolecule that has been known and used for several years ["Lipases" eds. H.L Bergstron and H. Brockman, Elsevier, Amsterdam, 1984] in chiral resolution through hydrolysis, not only of compounds directly related to its natural substrate - such as the glycerine esters of long-chain fatty acids - but also of racemic mixtures of chiral compounds, due to its capacity for carrying out the enantioselective hydrolysis of all types of racemic esters, whereby it finds chirality in alcohol as well as in acids. Patent application P9601847 describes its immobilization on inorganic supports.

PPL is increasingly being used everyday for the enzymatic resolution of highly diverse racemic mixtures, either by hydrolysis or, to a lesser extent, by transesterification, albeit for the time being it has not been applied to the production of biodiesel. The use of this enzyme is due to its easy accessibility (on the international market, it is commercialized by several suppliers), its high degree of stability - in addition to its not requiring the use of co-factors - but above all due to its very low price, when compared to other lipases (Table 1), since 10⁶ U may be acquired for only € 0.5, as per the Sigma catalogue.

**Table 1**

| Different types of lipases used in biodiesel production and the estimated cost of these | | |
|---|---|---|
| Lipase | 10⁶ U /€ | Bibliographic Reference |
| **Candida Antarctica** | 1155.0 | Y. Xu et al. Biotechnology Letters 25 (2003) 1239. |
| **Candida Cilindracea** | 21816.0¹ | P.V. Lara et al, Enzyme and Microbial Technology 34 (2004) 270. |
| **Candida Rugosa** | 1.4 | Y.Y. Linko et al, J. Am. Chem. Oil Soc., 71 (1994) 1411. |
| **Candida sp** | --- | M. Mittelbach et al, Fat Sci. & Technol., 92 (1990) 145. |
| **Chromobacteri um viscosum** | 17100.0 | S. Shah et al, Energy & Fuels,18 (2004) 154. |
| **Cryptococus spp. S-2** | - | N.R. Kamini et al, Process Biochemistry, 37 (2001) 405. |
| **Mucor miehei** | 92.8 | D.De Oliveira et al, Appl. Bioche. Biotechnol., 113 (2004)771. |
| **Pseudomonas cepacia** | 4392.0¹ | A. Hsu et al, Biotechnol.Appl. Biochem., 36 (2002)181. |
| **Pseudomonas fluorescens** | 4660.0 | M.M. Soumanou et al, Enzyme and Microbial Technology,33 (2003) 97. |
| **Rhizomucor miehei** | 20.5 | S. Al-Zuhair, Biotechnol Prog., 21 (2005) 1442 |
| **Rhizopus oryzae** | 106.0¹ | K. Ban et al, Biochem. Engineering J., 8 (2001) 39. |
| **Thermomyces lanuginosus** | 4.1 | Y. Xu et al, Biocatal. Biotransform., 22 (2004) 45. |

| | | |
|---|---|---|
| ¹Fluka catalogue. | | |

Summarizing, in the prior art has not been disclosed the use of pig pancreatic lipase (PPL) due to its 1.3 selective characteristic. This fact could be analysed in several documents wherein it is not reached a complete reaction (with a 50-80% of the conversion in biodiesel) despite the relative high concentrations of PPL (0.5g PPL for 5.7mmol of sunflower oil). Nevertheless, the use of a pH=10 reduce the use of PPL for the conversion of 100mmol of sunflower oil, multiplying the efficiency per 100.

Other prior art documents discloses that the PPL is inactive in a free form and extremely few active in an immobilized form. The PPL is the most economic lipase, but at the moment has not been considered as utilizable for biodiesel production, but using a complete transesterification, not esterification, under determined conditions of pH it is possible. This point has to be considered the technical problem that the present patent application solves.

The present invention aims to resolve the inconveniences that biodiesel production currently implies and improve its yield in all aspects.

DESAI P D ET AL: "Alcoholysis of salicornia oil using free and covalently bound lipase onto chitosan beads" FOOD CHEMISTRY, ELSEVIER LTD, NL LNKD-DOI:10.1016/J.FOODCHEM.2004.12.030, vol. 95, no. 2, 1 March 2006 (2006-03-01), pages 193-199, XP025130211 ISSN: 0308-8146; discloses the alcoholysis of salicornia oil, mediated by free and immobilized lipase, at 25°C using methanol in hexane and acetone.

SHAH ET AL.: 'Biodiesthe preparation by lipase catalyzed transesterification of Jatropha oil' ENERGY & FUELS, AMERICAN CHEMIAL SOCIETY vol. 18, 2004, pages 154 - 159,discloses the transesterification reaction of Jatropha oil in a solvent-free system to produce biodiesel using three different lipases (*Chromobacterium viscosum, Candida rugosa,* and Porcine pancreas).

YESILOGLU ET AL. 'Immobilized lipase catalyzed ethanolysis of sunflower oil' JOURNAL OF THE AMERICAN OIL CHEMISTS' SOCIETY vol. 81, 2004, pages 157 - 160, XP008131147 discloses the ethanolysis of sunflower oil catalyzed by an immobilized 1,3-specific porcine pancreatic lipase. The reaction is carried out in a oil/ethanol molar ratio of 1:3, at 45° C.

### DETAILID DESCRIPTION OF THE INVENTION

The present invention as defined in the claims refers to a procedure for producing biodiesel characterized in that it involves carrying out a transesterification reaction on an initial product selected from among one or more oils, one or more fats, and mixtures of these, in the presence of pig pancreatic lipase as a biocatalyst, at a pH of at least 8, preferably at least 10.

The pig pancreatic lipase may be free or attached to an organic or inorganic support; for example, it may be adsorbed on an organic or inorganic support.

Preferably, the PPL is used in immobilized form by chemical attachment of the enzyme to an inorganic solid that acts as support, allowing for its easy recovery at the end of the process and its subsequent reuse. The pig pancreatic lipase is present in free or attached form in a proportion of 1/1000 with respect to the weight of the oil or fat used, making it possible to convert 1 mole of oil in 24 hours per gram of PPL.

It is possible to use any type of pig pancreatic lipase; for instance, lipase selected from a choice of purified commercial industrial PPL, an enzymatic preparation containing it - such as for instance Sigma's Pancreatin, and a PPL preparation extracted directly from pig pancreas.

In the invention procedure, the said initial product - oil or fat - may be selected from a choice of one or more vegetable oils, one or more vegetable fats, one or more animal oils, one or more animal fats and mixtures of these.

In addition, said fat or oil may be a choice between pure oil or fat and used oil or fat. As per a specific assay, said initial product is chosen from among one or more glycerine esters. This term shall refer hereinafter to mono-, di- or triesters; i.e., one or more monoglycerides, diglycerides, triglycerides or mixtures of these.

The transesterification reaction of said oil or said fat is carried out with one or more short-chain alcohols; i.e., having the formula CₙH₂ₙ₊₂O, in which n ≤ 6.

The alcohols of the formula CₙH₂ₙ₊₂O are methanol, ethanol, 1-propanol, 2-propanol, 1-butanol, 2-butanol, 2-methyl-2-propanol, 1-pentanol, and mixtures of these.

Said transesterification reaction is preferably carried out at normal atmospheric pressure, at a temperature of between 20-70° C, preferably between 20-50° C, and at a pH of between 8 and 13, most preferably between 10 and 13. This pH is preferably obtained through the addition of a base chosen from among the following: NaOH, KOH, NH₄OH, Na₂CO₃, NaHCO₃, K₂CO₃, KHCO₃, and a mixture of these. The base is in the form of a concentrated aqueous solution acting as a co-adjuvant.

The transesterification reaction may be carried out either in an agitated-tank reactor or in a flow reactor.

As per a specific assay of the procedure, the transesterification reaction is carried out by mixing equimolar amounts of oil or fat and alcohol, thus preferably obtaining biodiesel. Preferably, when diglycerides or triglycerides are used as initial products and equimolar amounts of these are used with the alcohol, only biodiesel is obtained.

As per an additional specific assay of the procedure, one or more triglycerides are used as initial products and the transesterification is carried out subject to control of the operating conditions, with a molar proportion of 1/2 in triglycerides to alcohol.

Another way of controlling the reaction to preferably or exclusively obtain biodiesel is to control conditions such as temperature and reaction time, so that the transesterification does not reach its end. By mixing equimolar amounts of oil or fat and alcohol - for example, of diglycerides and/or triglycerides, subject to temperature control for a pre-determined time - biodiesel is obtained in a single step. This is a concrete case of consecutive reaction that yields - if glycerine esters are used as initial product - a mixture consisting of one mole of monoglyceride for every two moles of esters from the alcohol used, and thus, without glycerine, making it possible to directly use this mixture (biodiesel) in an appropriate (diesel) engine.

Hence, as per a specific additional assay of the procedure, the transesterification reaction is carried out in the course of a controlled reaction time so that the transesterification does not reach its end.

As per a specific additional assay of the procedure, the transesterification reaction is carried out with a mixture of equimolar amounts of oil or fat and alcohol, whereby the reaction is maintained for a period of between 8 and 72 hours, preferably between 8 and 48 hours, at a temperature of between 20° and 70° C, preferably between 20° C and 50° C.

As per a specific additional assay, one or more glycerine esters are used as initial products and the transesterification takes place subject to control of the operating conditions, with an equimolar proportion of glycerine esters and alcohol, thus obtaining biodiesel.

As per a specific additional assay, one or more glycerine esters are used as initial products and the transesterification takes place subject to control of the operating conditions, with a volumetric proportion of glycerine esters/alcohol greater than or equal to 1. Preferably, the volumetric proportion of glycerine esters/alcohol is greater than or equal to 10.

A specific additional assay refers to a procedure in which triglycerides are used as initial products and the transesterification takes place subject to control of the operating conditions, with equimolar amounts of triglycerides and alcohol for a period of between 8 and 30 hours, at a temperature of between 20° and 70° C, preferably between 20° and 50° C.

A particularly preferred assay for obtaining biodiesel is one in which the transesterification reaction is carried out on an oil selected from a choice between pure sunflower oil and used sunflower oil, with an alcohol selected from a choice of either methanol, ethanol 1-propanol, 2-propanol 1-butanol, 2-butanol, 2-methyl-2-propanol, or 1-pentanol in the molar proportion of 1/3, and using free or immobilized PPL. Most preferably, a transesterification reaction is carried out on sunflower oil using ethane, with a volumetric proportion of at least 8/1 of oil/ethanol, at a temperature of between 35° C and 50° C, preferably at 45°C, and at a pH of between 9 and 13, most preferably at pH=12.

A particularly preferred additional assay for obtaining biodiesel is one in which a transesterification reaction is carried out on sunflower oil with ethyl alcohol using free-form PPL in an agitated-tank reactor, discontinuously, at 45° C and pH = 12.

On another hand, as per an additional assay, when the procedure of the present invention is carried out such that it includes control over the conditions of transesterification, with a volumetric proportion of alcohol/glycerine esters such that there is an excess of alcohol (for example, greater than or equal to 1/10), along with the mixture of alcohol esters making up the biodiesel, glycerine is also obtained as a product of the reaction. The glycerine obtained that is dissolved in the alcohol phase can be separated from the biodiesel by decantation. The glycerine obtained that is dissolved in the alcohol phase can be separated from the alcohol by distilling said alcohol. In the case concerned, glycerine of an optimum purity (usable for pharmaceutical purposes and expensive) as per this assay of the procedure can be obtained, departing, for instance, from a 2/1 volumetric mixture of oil/alcohol, and operating under certain temperature conditions for a time frame in excess of that required to exclusively obtain biodiesel, towards the purpose of bringing the reaction to its end. Thus, a mixture of three moles of esters from the alcohol used is obtained for every glycerine mole, which will dissolve in the alcohol phase because it operates in an excess of alcohol. The alcohol phase will separate by decantation from the mixture of the esters from the alcohol used (methyl, ethyl, etc.).

Thus, when one or more glycerine esters are used as initial products and transesterification is carried out with amounts of alcohol that are superior to stoichiometric levels in relation to the glycerine esters for a reaction time that is sufficient for transesterification to come to an end, glycerine is obtained as a product of the reaction.

When one or more glycerine esters are used as initial products and transesterification is carried out with a volumetric proportion of alcohol/glycerine esters in which there is an excess of alcohol - for example, of 10/1 or more - for a reaction time that is sufficient for transesterification to come to an end, glycerine is obtained as a product of the reaction.

In addition, the present invention refers to the use of the procedure for obtaining biodiesel previously defined, as well as the use of the procedure defined describing the adequate characteristics for obtaining glycerine.

In the case of interest - obtaining high-purity glycerine - the method may be applied with clear advantages, since it would suffice to use a greater amount of alcohol than the stoichiometric amount, which would serve to obtain two phases. The glycerine is found in the denser of the two, along with the alcohol. The glycerine will be obtained very easily by the simple distillation of the alcohol. The absence of alkaline salts in the enzymatic process determines the purity of the glycerine obtained, without the need for subsequent steps to purify it.

On another hand, as per the procedure of the invention and through control of operating time and conditions (pH, temperature, etc.) the mixture of esters and the pertinent monoglycerides can be obtained as a product of the reaction practically without generating glycerine, eliminating the need to dispose of it by means of washing, and thus simplifying the process of obtaining biodiesel, reducing the environmental impact of the process at the same time.

The present procedure for obtaining biodiesel has many advantages, given that, in general, enzymatic reactions, as has been indicated, are experimentally much more accessible than catalytic reactions. Hence, these take place under milder conditions of reaction: normal atmospheric pressure, temperature (30°-50° C) and pH within the range of 8-13, preferably 9-13, spelling out a simplification in terms of instruments required and an ease of manipulation that, in the end, will redound to a reduction in production costs.

Contrary to what happens in catalytic processes occurring in an excess of alcohol - normally (1:3) - in transesterifications using lipases, it is possible to operate with molar proportions of oil - alcohol (1:1) without affecting process performance, ensuring that biodiesel is obtained in a single step from the equimolar mixture of the oil and the alcohol. Moreover, apart from being able to employ any kind of oil or fat (pure or used), any short-chain alcohol may be employed (methanol, ethanol, propanol, etc.), in contrast to the catalytic process that works adequately only with methanol. Lastly, the mild character of the process with which transesterification with lipases is carried out makes it possible to monitor the moment at which the reaction must be detained, so as to obtain the most adequate mixture of fatty acid esters and their corresponding monoglycerides, since this biodiesel will contain practically no glycerine.

The procedure for the production of biodiesel described may be applied in practically any of the (still rare) installations currently existing, or may be carried out by using enzymatic reactors commercially available on the market for other purposes.

Hence, given the virtual character of enzymatic catalysis (both in free and immobilized form), this would imply a marked simplification in biodiesel production from the economic as well as the environmental point of view, since this type of catalysts would make application possible in continuous large-scale processes as well as discontinuous processes on a smaller scale, since everyone profits from the reduction of the post-biodiesel synthesis washing process to a minimum, a process that today represents the most uneconomical factor of the process. Without this handicap, it is possible to imagine that certain agricultural cooperatives would undertake to produce their own biodiesel, since they generally have the raw materials at their disposal, and the technology of the reactor required and a commercial decolouration (or interesterification) plant would be sufficient once the problem of generating waste, and, principally, the high cost of the lipases that have been investigated up to the present are resolved.

Likewise, the procedure presented contemplates possible application not only using any commercialized industrial PPL, either purified or present in commercially available, low-cost enzymatic preparations containing lipases - such as for instance Sigma's Pancreatin - but also even using those PPL preparations directly extracted from pig pancreas, including variants that affect the parameters determining conditions of operation: temperature, pH, alcohol type (methanol, ethanol, isopropanol, etc.) and oil (sunflower, soy, etc., and used oil) and their molar proportions.

The procedure of the present invention makes it possible to obtain the product known as biodiesel in a very simple way that is at the same time economically profitable through the reaction of any oil or fat (pure or used) with any short-chain alcohol (methanol, ethanol, propanol, etc.) in the desired volumetric proportion, preferably oil/alcohol > 1/1, (and preferably 10/1, which approximately corresponds to the molar proportion of 1/3), at normal atmospheric pressure, at a temperature of between 20°-70° C, preferably 20°-50 °C, at a pH within the range of 8-13, preferably 10-13 (obtained through the addition of any base: NaOH, KOH, NH₄OH, Na₂CO₃, NaHCO₃, K₂CO₃, KHCO₃ etc., or mixture of these), and with an amount of free or immobilized PPL in the order of 1/1000 with respect to the weight of the oil used.

The process set forth by the present invention presents some general characteristics that are similar to those of the processes cited in Table 1, with the significant difference that it deals with the use of lipase from pig pancreas; and it presents essential advantages, such as: its high degree of stability, easy access on the international market, the fact that it does not require the use of co-factors, and, above all, its low cost (compare ∈ 0.5 per 10⁶ U with the values reflected in Table 1 for other microbial lipases), which may imply decisive economic advantages in its application to the production of biodiesel.

The following examples illustrate the practical application of the present invention.

### MODES OF APPLYING THE INVENTION

### Example 1: Application of the method employing free-form PPL, with different molar proportions of oil/ethanol.

Table 2 reflects the results obtained with free-form PPL operating discontinuously in an agitated-tank reactor with sunflower oil and 96% ethyl alcohol at 45°C, pH = 12 and different reaction times, indicating the relative amounts of ethyl esters (fatty acid esters or FAE), monoglycerides (MG), diglycerides (DG) and triglycerides (TG). Distinction is made as to conversion (Conv.) and performance (Perf.) parameters as per the consideration given to the presence of DGs in the final biodiesel mixture. This is to say that the performance parameter reflects the proportion of esters and monoglycerides as desirable components of the biodiesel.

An HP 5890 series II gas chromatograph with an HT5 capillary column of 0.1 UM (25m x 0.32 mmm)SGE was used to arrive at this quantification, applying a program of gradually increasing temperatures that starts at 90°C up to 200°C at 5°C/minute, in which temperature is maintained for 10 minutes and heating starts again up to 350° C (at 10°C/ minute, said temperature being finally maintained for another ten minutes. Injector and detector temperatures are 380°C and 400°C, respectively.

The results obtained demonstrate that performance and conversion increase as the alcohol present in the reaction medium decreases (contrary to what happens in a process of catalysis in basic medium (with NaOH or KOH).

It may be noted in addition that the optimum results for not producing glycerine are obtained in equimolar ratios of oil/alcohol, which correspond (depending on the specific type of oil and alcohol) to the volumetric proportions of oil/alcohol that are greater than 1/1 and preferably greater than 15/1. From these results, it may be concluded that the optimum process makes it possible to obtain biodiesel in a single step, after the total conversion of the initial reagents, oil and alcohol, whereby the elimination of the excess alcohol is not necessary, as usually occurs with processes of catalysis in basic media.

**Table 2**

| Composition of the biodiesel obtained in the transesterification of 0.1 mol (120 mL) of sunflower oil with 0.1 g of PPL and different proportions of oil/ethanol 96% at 45° C, pH = 12 and different reaction times. | | | | | | | |
|---|---|---|---|---|---|---|---|
| ***Oil*/ *Alcohol (ml*/*ml)*** | ***Time (h)*** | ***FAE (%)*** | ***MG (%)*** | ***DC (%)*** | ***TG (%)*** | ***Perf. (%)*** | ***Conv. (%)*** |
| 1/1 | 10 | 32.8 | 2.5 | 27.9 | 36.8 | 18.2 | 41.2 |
| " | 24 | 35.7 | 3.8 | 28.1 | 32.4 | 21.3 | 46.1 |
| 2/1 | 10 | 47.1 | 0 | 50.4 | 8.6 | 24.8 | 83.8 |
| " | 24 | 46.0 | 0 | 47.3 | 6.8 | 28.9 | 86.7 |
| 4/1 | 10 | 29.5 | 0 | 70.5 | 0 | 17.4 | 100 |
| " | 24 | 32.3 | 0 | 67.7 | 0 | 19.3 | 100 |
| 8/1 | 10 | 27.8 | 0 | 72.2 | 0 | 16.2 | 100 |
| " | 24 | 35.3 | 0 | 64.7 | 0 | 21.5 | 100 |
| 15/1 | 10 | 40.6 | 0 | 54.9 | 4.4 | 25.0 | 91.5 |
| " | 24 | 46.4 | 0 | 53.6 | 0 | 30.3 | 100 |

### Example 2: Application of the method employing free-form PPL with pure or used oil and different types of alcohol

Table 3 reflects the results corresponding to the behaviour of PPL with pure sunflower oil or used oil and with different short-chain alcohols: methanol (MeOH), ethanol 96% (EtOH), 1-propanol (1-PrOH), 2-propanol (2-PrOH), 1-butanol (1-BuOH), 2-butanol (2-BuOH), 2-methyl-2-propanol (t-BuOH) and 1-pentanol (1-PeOH).

**Table 3**

| Composition of the biodiesel obtained in the transesterification of 0.1 mol (120 mL) of oil (sunflower oil or used oil) with 0.1 g of PPL and different alcohols, in an 8/1 volumetric proportion of oil/alcohol [molar ratio 1/3], at 45° C, pH = 12 and different reaction times. | | | | | | | | |
|---|---|---|---|---|---|---|---|---|
| ***Alcohol*** | ***Oil*** | ***t (h)*** | ***FAE (%)*** | ***MG (%)*** | ***DG (%)*** | ***TG (%)*** | ***Perf. (%)*** | ***Conv (%)*** |
| MeOH | Pure | 24 | 65.1 | 30.0 | 4.9 | 0 | 90.9 | 100 |
| EtOH | Pure | 10 | 27.8 | 0 | 72.2 | 0 | 16.2 | 100 |
| EtOH | Pure | 24 | 35.3 | 0 | 64.7 | 0 | 21.5 | 100 |
| EtOH | Used | 5 | 20.7 | 0 | 61.5 | 17.8 | 10.7 | 71.3 |
| EtOH | Used | 9 | 30.1 | 0 | 54.8 | 15.2 | 16.5 | 73.9 |
| EtOH | Used | 24 | 11.6 | 0 | 73.5 | 14.9 | 11.6 | 75.5 |
| 1-PrOH¹ | Pure | 16 | 56.9 | 13.5 | 29.6 | 0 | 56.0 | 100 |
| 1-PrOH¹ | Pure | 22 | 57.8 | 15.5 | 26.7 | 0 | 59.6 | 100 |
| 1-PrOH | Pure | 24 | 58.9 | 17.6 | 23.5 | 0 | 63.6 | 100 |
| 2-PrOH¹ | Pure | 16 | 22.9 | 0 | 77.1 | 0 | 13.5 | 100 |
| 2-PrOH¹ | Pure | 24 | 19.6 | 0 | 80.4 | 0 | 11.4 | 100 |
| 2-PrOH | Pure | 28 | 56.4 | 12.5 | 31.1 | 0 | 54.2 | 100 |
| 1-BuOH | Pure | 16 | 49.3 | 7.1 | 35.0 | 8.6 | 39.7 | 81.8 |
| 1-BuOH | Pure | 24 | 47.5 | 5.4 | 36.8 | 10. 3 | 36.1 | 78.7 |
| 2-BuOH | Pure | 13 | 59.6 | 19.1 | 21.3 | 0 | 67.1 | 100 |
| 2-BuOH | Pure | 22 | 65.7 | 31.4 | 2.9 | 0 | 94.9 | 100 |
| t-BuOH | Pure | 24 | 52.3 | 14.0 | 24.3 | 9.4 | 49.2 | 78.9 |
| 1-PeOH | Pure | 24 | 58.9 | 18.0 | 23.2 | 0 | 65.5 | 100 |

| | | | | | | | | |
|---|---|---|---|---|---|---|---|---|
| ¹Ratio 3.2/1 | | | | | | | | |

In contrast to basic catalysis, any alcohol may be used in the transesterification. It may also be observed how the use of recycled oil brings about a slight decrease in conversion, as has been borne out in the use of other lipases.

### Example 3: Application of the method employing a commercial Sigma preparation called Pancreatin, which contains PPL, as biocatalyst, together with other enzymes.

The results obtained upon carrying out the transesterification reaction of sunflower oil with ethanol using different amounts of Pancreatin - a Sigma commercial preparation that contains PPL along with other extra-cellular enzymes - are reflected in Table 4, which specifies the experimental conditions of the reaction.

**Table 4**

| Composition of the biodiesel obtained in the transesterification of 0.1 mol (120 mL) of sunflower oil with different amounts of Pancreatin, operating in an 8/1 volumetric proportion of oil/alcohol, at 45° C, pH = 12 and different reaction times. | | | | | | | |
|---|---|---|---|---|---|---|---|
| ***Pancreatin (g)*** | ***t (h)*** | ***FAE (%)*** | ***MG (%)*** | ***DG (%)*** | ***TG (%)*** | ***Perf. (%)*** | ***Conv. (%)*** |
| 0.1 | 24 | 11.6 | 0 | 73.5 | 14.9 | 5.8 | 76.7 |
| " | 48 | 14.1 | 0 | 78.0 | 8.0 | 7.3 | 87.0 |
| " | 72 | 17.2 | 0 | 82.8 | 0 | 9.5 | 100 |
| 0.2 | 24 | 52.0 | 4.9 | 42.6 | 0.6 | 40.0 | 98.7 |
| 0.5¹ | 23 | 62.0 | 28.6 | 5.3 | 0 | 90.1 | 100 |
| 0.8 | 20 | 56.4 | 13.1 | 30.6 | 0 | 54.0 | 100 |

| | | | | | | | |
|---|---|---|---|---|---|---|---|
| ¹4.1% of glycerine is obtained | | | | | | | |

Despite the need for a greater weight of the enzyme preparation than occurs with purified PPL, the practical application of the method to the production of biodiesel is possible, since the purified PPL may be used in free form as well as immobilized by any procedure or on any support.

### Example 4: Application of the method employing PPL immobilized onto an inorganic support

Tables 5 and 6 reflect the results obtained on applying PPL immobilized onto an inorganic solid - a commercially available sepiolite - by applying a method described in P9601847 for the purpose.

To immobilize the enzyme, it is sufficient to put the amount of the inorganic solid previously activated as described in the cited patent (0.5 g) in contact with the amount of the chosen PPL (0.04 g) in the reaction beaker (50 mL) with 6 mL of ethanol. Shake manually and keep in refrigeration for 24 hours, so that the covalent interaction of the PPL lysine residues with the activated groups (aldehyde carbonyls) can take place.

Next, with the help of another 6 mL of ethanol, the non-immobilized PPL is extracted (Table 5, No. 0^{b}). The catalytic activity of this solution provides the value of the amount of PPL that has not been covalently immobilized, and the comparison of this value with the activity of the immobilized PPL (Table 5, No. 4) and free PPL (Table 5, No. 0^{a}) makes it possible to determine the quantity of immobilized enzyme and its effectivity. Table 6 proceeds with the reutilization of the same immobilized biocatalyst in carrying out a new series of reactions where methanol is used.

**Table 5.**

| | | | | | | | | | | |
|---|---|---|---|---|---|---|---|---|---|---|
| Composition of the biodiesel obtained in the transesterification of 0.01 mol (12 mL) of sunflower oil and 6 mL of absolute ethanol (EtOH) using free-form PPL (0.01 g) and immobilized PPL (0.5 g solid, 0.04 g of lipase), different reaction times, and different pH and temperature values. | | | | | | | | | | |

| ***No.*** | ***t (h)*** | ***T (°C)*** | ***pH*** | ***FAE (%)*** | ***MG (%)*** | ***DG (%)*** | ***TG (%)*** | ***GLY (%)*** | ***Perf. (%)*** | ***Conv. (%)*** |
|---|---|---|---|---|---|---|---|---|---|---|
| 0^{a} | 24 | 40 | 10 | 11.4 | 0 | 47.9 | 40.8 | 0 | 5.1 | 42.7 |
| 0^{b} | 24 | 40 | 10 | 10.2 | 0 | 48.1 | 41.8 | 0 | 4.5 | 41.8 |
| 1 | 24 | 25 | 10 | 15.4 | 0 | 32.3 | 52.3 | 0 | 6.8 | 28.3 |
| 2 | 24 | 30 | 10 | 9.1 | 0 | 50.6 | 40.4 | 0 | 4.0 | 43.7 |
| 3 | 24 | 35 | 10 | 12.6 | 0 | 46.9 | 40.5 | 0 | 5.7 | 42.7 |
| 4 | 24 | 40 | 10 | 17.4 | 0 | 43.6 | 39.0 | 0 | 8.1 | 43.3 |
| 5 | 70 | 40 | 10 | 41.2 | 0 | 58.8 | 0.0 | 0 | 26.1 | 100 |
| 6 | 24 | 45 | 10 | 10.4 | 0 | 46.8 | 42.8 | 0 | 4.6 | 40.5 |
| 7 | 24 | 50 | 10 | 22.7 | 0 | 66.6 | 10.7 | 0 | 12.3 | 82.0 |
| 8 | 72 | 25 | 12 | 60.2 | 24.3 | 12.0 | 0 | 3.5 | 78.6 | 100 |
| 9 | 23 | 30 | 12 | 58.9 | 18.3 | 22.8 | 0 | 0 | 63.8 | 100 |
| 10 | 24 | 35 | 12 | 54.3 | 8.9 | 36.8 | 0 | 0 | 46.8 | 100 |
| 11 | 30 | 40 | 12 | 53.6 | 16.2 | 21.7 | 8.6 | 0 | 51.6 | 80.9 |
| 12 | 24 | 45 | 12 | 60.0 | 20.6 | 19.4 | 0 | 0 | 68.3 | 100 |
| 13 | 24 | 40 | 8 | 61.3 | 23.0 | 15.7 | 0 | 0 | 73.7 | 100 |
| 14 | 24 | 40 | 9 | 47.7 | 0 | 51.7 | 0.5 | 0 | 31.4 | 98.9 |

| | | | | | | | | | | |
|---|---|---|---|---|---|---|---|---|---|---|
| ^{a}Free enzyme (0.01 g). ^{b}Free enzyme, in the filtrate, after immobilization. | | | | | | | | | | |

**Table 6**

| | | | | | | | | | | |
|---|---|---|---|---|---|---|---|---|---|---|
| Composition of the biodiesel obtained in the transesterification of 0.01 mol (12 mL) of sunflower oil and 6 mL de methanol (MeOH), using the same biocatalyst already employed in Table 5, immobilized PPL (0.5 g solid, 0.04 g of lipase), at different reaction times, with different pH and temperature values. | | | | | | | | | | |

| ***No .*** | ***t (h)*** | ***T (°C)*** | ***pH*** | ***FAE (%)*** | ***MG (%)*** | ***DG (%)*** | ***TG (%)*** | ***GLY (%)*** | ***Perf. (%)*** | ***Conv (%)*** |
|---|---|---|---|---|---|---|---|---|---|---|
| 15 | 24 | 25 | 12 | 61.2 | 21.7 | 17.1 | 0 | 0 | 70.9 | 100 |
| 16 | 24 | 30 | 12 | 61.3 | 21.9 | 16.8 | 0 | 0 | 71.4 | 100 |
| 17 | 24 | 35 | 12 | 57.8 | 28.9 | 0 | 0 | 13.3 | 100 | 100 |
| 18 | 24 | 40 | 12 | 62.5 | 31.2 | 0.3 | 0 | 6.1 | 99.4 | 100 |
| 19 | 24 | 45 | 12 | 60.8 | 30.2 | 0.3 | 0 | 8.7 | 99.3 | 100 |
| 20 | 72 | 25 | 10 | 55.7 | 18.9 | 17.1 | 0 | 8.4 | 68.6 | 100 |
| 21 | 30 | 30 | 10 | 60.0 | 30.0 | 0 | 0 | 10.1 | 100 | 100 |
| 22 | 26 | 35 | 10 | 55.4 | 26.2 | 2.5 | 0 | 15.8 | 94.2 | 100 |
| 23 | 22 | 40 | 10 | 65.5 | 32.7 | 0.1 | 0 | 1.7 | 99.8 | 100 |
| 24 | 24 | 45 | 10 | 60.3 | 30.2 | 0 | 0 | 9.5 | 100 | 100 |

After these two series of experiments done over a period of two months of successive use, at different temperatures, pH, using different alcohols, etc., the system maintains its initial catalytic activity. In fact, other reactions were carried out that either complete and/or repeat the reactions already set forth in Tables 5 and 6, investigating the influence of different volumetric proportions of oil/ethanol and using other alcohols, whereby it was ascertained that the enzymatic catalyst employed kept up its initial activity after more than 40 successive experiments and four months of continued use.

The results reflected in Table 5 demonstrate that 75% of the PPL was covalently immobilized to the support, while at the same time the efficiency of this enzyme was remarkably reduced (1/3). This notwithstanding, the immobilized enzyme suffers minimal influence from the reaction medium in comparison with the free enzyme (Tables 2 and 3) and, above all, shows a great resistance to both deactivation and leaching (or enzyme loss). It must be borne in mind that all the experiments described in Tables 5 and 6 were carried out successively for 2 months using the same catalyst, and that after more than 40 successive experiments and four months of continued use, the catalyst with covalently immobilized PPL still kept up its initial activity.

Despite the loss of PPL efficiency following covalent immobilization to the inorganic solid, the application of this immobilizing system offers diverse advantages, such as reuse, ease of separation, and, above all, the extraordinary stability that the PPL acquires after immobilization with respect to the more usual reaction parameters: temperature, pH, etc., making a permanent control of the composition of the ester mixture making up the biodiesel possible, and thus making it possible to decide to produce a glycerine-free compound or to opt to obtain it for subsequent separation (Table 6, No. 17, 21, 22 and 24), as interests dictate.

## Claims

1. Procedure for the production of biodiesel **characterized in that** it involves carrying out the transesterification reaction of an initial product selected from a choice of one or more oils, one or more fats, and mixtures of these, with one or more alcohols having the formula CₙH₂ₙ₊₂O, in which n < 6 wherein said alcohols are selected from a choice between methanol, ethanol, 1-propanol, 2-propanol, 1-butanol, 2-butanol, 2-methyl-2-propanol, 1-pentanol and mixtures of these, in the presence of pig pancreatic lipase as a biocatalyst in a proportion of 1/1000 with respect to the weight of the oil or fat, at a pH of at least 8.

2. Procedure for the production of biodiesel according to claim 1, **characterized in that** pH value is at least 10.

3. Procedure according to claim 1, **characterized in that** the pig pancreatic lipase is attached to an organic or inorganic support.

4. Procedure according to one of the foregoing claims, **characterized in that** the pig pancreatic lipase is selected from a choice between purified industrial PPL commercially available, a commercial enzymatic preparation containing it, and a preparation of PPL extracted directly from pig pancreas.

5. Procedure according to claim 1, **characterized in that** the said fat or oil is selected from a choice between a pure oil or fat and a used oil or fat and wherein said oils or fats are selected from a choice between vegetable oils, animal oils, vegetable fats, animal fats, and mixtures of these.

6. Procedure according to claim 1, **characterized in that** said initial product is selected from one or more glycerine esters.

7. Procedure according to any of the foregoing claims, **characterized in that** said transesterification reaction is carried out at normal atmospheric pressure, at a temperature of between 20°-70° C and with a pH of between 8 and 13 wherein said pH is obtained through the addition of a base in the form of an aqueous solution selected from a choice between NaOH, KOH, NH₄OH, Na₂CO₃, NaHCO₃, K₂CO₃, KHCO₃ and a mixture of these.

8. Procedure according to any of the foregoing claims, **characterized in that** said transesterification reaction is carried out in a reactor selected from a choice between an agitated-tank reactor and a flow reactor, mixing equimolar amounts of oil or fat and alcohol, during a controlled reaction time so that the transesterification does not reach its end.

9. Procedure according to claim 8, **characterized in that** the transesterification reaction is carried out on a mixture of equimolar amounts of oil or fat and alcohol, maintaining the reaction for a time period of between 4 and 72 hours, at a temperature of between 20° and 70° C.

10. Procedure according to any of foregoing claims 1 to 9, **characterized in that** one or more glycerine esters are used as initial products and the transesterification is carried out with control over the operating conditions, in a volumetric proportion of glycerine esters to alcohol that is greater than or equal to 10/1.

11. Procedure according to any of foregoing claims 1 to 8, **characterized in that** one or more triglycerides are used as initial products and the transesterification is carried out with control over the operating conditions, in a molar proportion of 1/2 of triglycerides with respect to alcohol.

12. Procedure according to one of previous claims 1 to 8, **characterized in that** triglycerides are used as initial product and the transesterification is carried out with control over the operating conditions, with equimolar amounts of triglycerides and alcohol, for a time period of between 4 and 72 hours, at a temperature of between 20° and 70° C.

13. Procedure according to claim 1, **characterized in that** one or more glycerine esters are used as initial products and the transesterification is carried out with stoichiometric amounts of glycerine esters and alcohol, with a volumetric proportion of alcohol to glycerine esters in a ratio equal or greater than 1/10, for a reaction time that is sufficient for the transesterification to reach its end, obtaining glycerine as a product of the reaction; and wherein said glycerine is dissolved in the alcohol phase, separating the biodiesel by decantation or wherein said glycerine is dissolved in the alcohol phase, separating the alcohol by distillation of said alcohol.

14. Procedure according to claim 1, **characterized in that** the transesterification reaction is carried out on sunflower oil and ethanol in the molar proportion of 1/3 and, using free-form PPL, with a volumetric proportion of at least 8/1 in terms of oil to alcohol, in an agitated-tank reactor, discontinuously, at 45° C and pH = 12.

## Patentansprüche

1. Verfahren zur Herstellung von Biodiesel, **dadurch gekennzeichnet, dass** es die Durchführung der Umesterungsreaktion eines Ausgangsprodukts umfasst, ausgewählt aus einer Auswahl von einem oder mehreren Ölen, einem oder mehreren Fetten und Mischungen dieser, wobei ein oder mehrere Alkohole die Formel CₙH₂ₙ₊₂O aufweisen, wobei n < 6, wobei die Alkohole ausgewählt sind aus einer Auswahl zwischen Methanol, Ethanol, 1-Propanol, 2-Propanol, 1-Butanol, 2-Butanol, 2-Methyl-2-propanol, 1-Pentanol und Mischungen dieser, in Gegenwart von Schweine-Pankreas-Lipase als Biokatalysator in einer Proportion von 1/1000 mit Bezug auf das Gewicht des Öls oder des Fetts, bei einem pH-Wert von mindestens 8.

2. Verfahren zur Herstellung von Biodiesel nach Anspruch 1, **dadurch gekennzeichnet, dass** der pH-Wert mindestens 10 beträgt.

3. Verfahren nach Anspruch 1, **dadurch gekennzeichnet, dass** die Schweine-Pankreas-Lipase an einen organischen oder einen anorganischen Träger befestigt ist.

4. Verfahren nach einem der vorhergehenden Ansprüche, **dadurch gekennzeichnet, dass** die Schweine-Pankreas-Lipase ausgewählt ist aus einer Auswahl zwischen gereinigter industrieller PPL, die kommerziell erhältlich ist, einer kommerziellen enzymatischen Zubereitung, die sie enthält, und einer Zubereitung aus PPL, die direkt aus der Schweine-Pankreas extrahiert wird.

5. Verfahren nach Anspruch 1, **dadurch gekennzeichnet, dass** das Fett oder Öl ausgewählt ist aus einer Auswahl zwischen einem reinen Öl oder Fett und einem gebrauchten Öl oder Fett, und wobei die Öle oder Fette ausgewählt sind aus einer Auswahl zwischen pflanzlichen Ölen, tierischen Ölen, pflanzlichen Fetten, tierischen Fetten und Mischungen dieser.

6. Verfahren nach Anspruch 1, **dadurch gekennzeichnet, dass** das Ausgangsprodukt ausgewählt ist aus einem oder mehreren Glycerinestern.

7. Verfahren nach einem der vorhergehenden Ansprüche, **dadurch gekennzeichnet, dass** die Umesterungsreaktion bei normalem atmosphärischem Druck, bei einer Temperatur von zwischen 20 und 70 °C und mit einem pH-Wert von zwischen 8 und 13 durchgeführt wird, wobei der pH-Wert durch die Zugabe einer Base in Form einer wässrigen Lösung, ausgewählt aus einer Auswahl zwischen NaOH, KOH, NH₄OH, Na₂CO₃, NaHCO₃, K₂CO₃, KHCO₃ und einer Mischung dieser, erhalten wird.

8. Verfahren nach einem der vorhergehenden Ansprüche, **dadurch gekennzeichnet, dass** die Umesterungsreaktion in einem Reaktor durchgeführt wird, ausgewählt aus einer Auswahl zwischen einem Rührkesselreaktor und einem Strömungsreaktor, wobei äquimolare Mengen von Öl oder Fett und Alkohol während einer kontrollierten Reaktionszeit gemischt werden, so dass die Umesterung nicht zu Ende gebracht wird.

9. Verfahren nach Anspruch 8, **dadurch gekennzeichnet, dass** die Umesterungsreaktion in einer Mischung aus äquimolaren Mengen von Öl oder Fett mit Alkohol durchgeführt wird, wobei die Reaktion während eines Zeitraums von 4 bis 72 Stunden bei einer Temperatur von zwischen 20 und 70°C aufrechterhalten wird

10. Verfahren nach einem der vorhergehenden Ansprüche 1 bis 9, **dadurch gekennzeichnet, dass** ein oder mehrere Glycerinester als Ausgangsprodukte verwendet werden und die Umesterung bei einer Kontrolle der Betriebsbedingungen in einer volumetrischen Proportion von Glycerinester zu Alkohol durchgeführt wird, die größer als oder gleich 10/1 ist.

11. Verfahren nach einem der vorhergehenden Ansprüche 1 bis 8, **dadurch gekennzeichnet, dass** ein oder mehrere Triglyceride als Ausgangsprodukte verwendet werden und die Umesterung bei einer Kontrolle der Betriebsbedingungen in einer molaren Proportion von 1/2 von Triglyceriden mit Bezug auf Alkohol durchgeführt wird.

12. Verfahren nach einem der vorhergehenden Ansprüche 1 bis 8, **dadurch gekennzeichnet, dass** Triglyceride als Ausgangsprodukt verwendet werden und die Umesterung bei einer Kontrolle der Betriebsbedingungen mit äquimolaren Mengen von Triglyceriden und Alkohol während eines Zeitraums von zwischen 4 und 72 Stunden bei einer Temperatur von zwischen 20 und 70°C durchgeführt wird.

13. Verfahren nach Anspruch 1, **dadurch gekennzeichnet, dass** ein oder mehrere Glycerinester als Ausgangsprodukte verwendet werden und die Umesterung mit stöchiometrischen Mengen von Glycerinestern und Alkohol mit einer volumetrischen Proportion von Alkohol zu Glycerinestern in einem Verhältnis gleich oder größer als 1/10 während einer Reaktionszeit durchgeführt wird, die ausreichend ist, um die Umesterung zu Ende zu bringen, wobei Glycerin als Produkt der Reaktion erhalten wird; und wobei das Glycerin in der Alkoholphase gelöst wird, wodurch der Biodiesel durch Dekantierung abgeschieden wird, oder wobei das Glycerin in der Alkoholphase gelöst wird, wobei der Alkohol durch Destillierung des Alkohols abgeschieden wird.

14. Verfahren nach Anspruch 1, **dadurch gekennzeichnet, dass** die Umesterungsreaktion in Sonnenblumenöl und Ethanol in der molaren Proportion von 1/3 und, unter Verwendung von PPL in freier Form, mit einer volumetrischen Proportion von mindestens 8/1 hinsichtlich Öl zu Alkohol in einem Rührkesselreaktor diskontinuierlich bei 45 °C und pH = 12 durchgeführt wird.

## Revendications

1. Procédé pour la production de biodiesel **caractérisé en ce qu'**il réalise la réaction de transestérification d'un produit de départ sélectionné parmi une ou plusieurs huiles, une ou plusieurs graisses ou bien un mélange de celles-ci, avec un ou plusieurs alcools dont la formule est CₙH₂ₙ₊₂O, dans laquelle n <6, dans laquelle les alcools sont sélectionnés parmi le méthanol, l'éthanol, 1-propanol, 2-propanol, 1-butanol, 2-butanol, 2-méthyl-2-propanol et 1-pentanol et des mélanges de ces derniers, en présence de la lipase pancréatique de porc comme biocatalyseur, dans une proportion de 1/1000 par rapport au poids de l'huile ou de la graisse et à un pH d'au moins 8.

2. Procédé pour la production de biodiesel selon la revendication 1, **caractérisé en ce que** le pH est de 10 au moins.

3. Procédé selon la revendication 1, **caractérisé en ce que** la lipase pancréatique de porc est fixée sur un support organique ou inorganique.

4. Procédé selon l'une des revendications précédentes, **caractérisé en ce que** la lipase pancréatique de porc est sélectionnée parmi des PLL purifiés disponibles sur le marché, une préparation enzymatique disponible commerciale la contenant et une préparation de PPL extrait directement du pancréas du porc.

5. Procédé selon la revendication 1, **caractérisé en ce que** cette graisse ou cette huile sont sélectionnées parmi une huile ou une graisse pure et une huile ou une graisse utilisée, ces huiles ou graisses étant sélectionnées parmi des huiles végétales, des huiles animales, des graisses végétales, des graisses animales et des mélanges de celles-ci.

6. Procédé selon la revendication 1, **caractérisé en ce que** ce produit de départ est sélectionné parmi un ou plusieurs esters de glycérine.

7. Procédé selon l'une des revendications précédentes **caractérisé en ce que** cette réaction de transestérification est faite à une pression atmosphérique normale, à une température comprise entre 20-70 °C et à un pH compris entre 8 y 13, dans lequel ce pH est obtenu à travers l'addition d'une base sous forme de solution aqueuse sélectionnée parmi NaOH, KOH, NH₄OH, Na₂CO₃, NaHCO₃, K₂CO₃, KHCO₃ et un mélange de ceux-ci.

8. Procédé selon l'une des revendications précédentes **caractérisé en ce que** cette réaction de transestérification est faite dans un réacteur sélectionné parmi un réacteur agité et un réacteur à flux, en mélangeant des quantités équimolaires d'huile ou de graisse et d'alcool, pendant un temps de réaction contrôlé pour que la transestérification n'arrive pas jusqu'au bout.

9. Procédé selon la revendication 8, **caractérisé en ce que** la réaction de transestérification est faite dans un mélange de quantités équimolaires d'huile ou de graisse et d'alcool, en maintenant la réaction pendant une durée comprise entre 4 et 72 heures à une température comprise entre 20 °C et 70 °C.

10. Procédé selon l'une quelconque des revendications 1 à 9 **caractérisé en ce que** l'on utilise un ou plusieurs esters de glycérine comme des produits de départ et la transestérification est effectuée par le biais d'un contrôle des conditions de l'opération et dans une proportion volumétrique des esters de glycérine par rapport à l'alcool, supérieure ou égale à 10/1.

11. Procédé selon l'une quelconque des revendications 1 à 8, **caractérisé en ce que** l'on utilise un ou plusieurs triglycérides comme produits de départ et la transestérification est effectuée au moyen d'un contrôle des conditions de l'opération et avec une proportion molaire de ½ de triglycérides par rapport à l'alcool.

12. Procédé selon l'une quelconque des revendications 1 à 8, **caractérisé en ce que** l'on utilise des triglycérides comme produit de départ et la transestérification est effectuée par le biais d'un contrôle des conditions de l'opération avec des quantités équimolaires de triglycérides et d'alcool pendant une durée comprise entre 4 et 72 heures à une température comprise entre 20 °C et 70 °C.

13. Procédé selon la revendication 1 **caractérisé en ce que** l'on utilise un ou plusieurs esters de glycérine comme produits de départ et la transestérification est effectuée avec des quantités stoechiométriques d'esters de glycérine et d'alcool, avec une proportion volumétrique d'alcool par rapport aux esters dans une relation supérieure ou égale à 1/10, pendant un temps de réaction suffisant pour que la transestérification arrive jusqu'au bout en obtenant de la glycérine comme produit de la réaction et dans lequel cette glycérine se dissout dans la phase alcoolique, en séparant le biodiesel par décantation, ou bien dans lequel cette glycérine se dissout dans la phase alcoolique, en séparant l'alcool par distillation de cet alcool.

14. Procédé selon la revendication 1, **caractérisé en ce que** la réaction de transestérification est faite dans de l'huile de tournesol et d'éthanol dans une proportion molaire de 1/3 et en utilisant le PPL sous forme libre, avec une proportion volumétrique d'au moins 8/1 en ce qui concerne l'huile par rapport à l'alcool, dans un réacteur agité, de façon discontinue, à 45 °C et pH = 12.
